(19) Europäisches Patentamt / European Patent Office / Office européen des brevets

(11) **EP 4 424 811 A1**

(12) **EUROPEAN PATENT APPLICATION**

(43) Date of publication:
**04.09.2024 Bulletin 2024/36**

(21) Application number: **24160068.3**

(22) Date of filing: **27.02.2024**

(51) International Patent Classification (IPC):
***C12M 1/12*** *(2006.01)*

(52) Cooperative Patent Classification (CPC):
**C12M 25/02;** C12M 25/14; C12M 25/18

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **28.02.2023 US 202363448907 P**

(71) Applicant: **Corning Incorporated**
**Corning, New York 14831 (US)**

(72) Inventor: **Goral, Vasiliy Nikolaevich**
**New York, 14870 (US)**

(74) Representative: **Elkington and Fife LLP**
**Prospect House**
**8 Pembroke Road**
**Sevenoaks, Kent TN13 1XR (GB)**

(54) **ROLLED CELL CULTURE SUBSTRATES FOR FIXED BED BIOREACTORS WITH CONTROLLED PERFUSION ZONES**

(57) A fixed-bed cell culture matrix formed from a rolled cell culture substrate is provided that includes a cell culture vessel having an inlet, an outlet, and an interior reservoir fluidly disposed in a fluid pathway between the inlet and the outlet. A cell culture matrix is disposed in the reservoir in a wound configuration around a winding axis. The cell culture matrix includes a structurally defined substrate from a substrate material defining a plurality of pores, the substrate material being designed for adhering cells thereto. A plurality of permeability zones are provided in the cell culture matrix, which include an opening in the substrate, where the opening being larger than a diameter of any of the plurality of pores. The plurality of permeability zones are arranged with a variable periodicity along a length of the substrate in the wound configuration.

FIGURE 9B

**Description**

**CROSS-REFERENCE TO RELATED APPLICATIONS**

This application claims the benefit of priority under 35 U.S.C. §119 of U.S. Provisional Application Serial No. 63/448,907 filed on February 28, 2023, the content of which is relied upon and incorporated herein by reference in its entirety.

**FIELD OF THE DISCLOSURE**

This disclosure generally relates to cell culture substrates in fixed bed configurations, as well as systems and methods for manufacturing such cell culture substrates. In particular, the present disclosure relates to fixed-bed cell culturing substrates in a rolled configuration with defined zones of controlled and variable porosity, as well as bioreactor systems incorporating such substrates, and methods of manufacturing cell culture substrate.

**BACKGROUND**

In the bioprocessing industry, large-scale cultivation of cells is performed for purposes of the production of hormones, enzymes, antibodies, vaccines, and cell therapies. Cell and gene therapy markets are growing rapidly, with promising treatments moving into clinical trials and quickly toward commercialization. However, one cell therapy dose can require billions of cells or trillions of viruses. As such, being able to provide a large quantity of cell products in a short amount of time is critical for clinical success.

A significant portion of the cells used in bioprocessing are anchorage dependent, meaning the cells need a surface to adhere to for growth and functioning. Traditionally, the culturing of adherent cells is performed on two-dimensional (2D) cell-adherent surfaces incorporated in one of a number of vessel formats, such as T-flasks, petri dishes, cell factories, cell stack vessels, roller bottles, and HYPERStack® vessels. These approaches can have significant drawbacks, including the difficulty in achieving cellular density high enough to make it feasible for large scale production of therapies or cells.

Alternative methods have been suggested to increase volumetric density of cultured cells. These include microcarrier cultures performed in stir tanks. In this approach, cells that are attached to the surface of microcarriers are subject to constant shear stress, resulting in a significant impact on proliferation and culture performance. Another example of a high-density cell culture system is a hollow fiber bioreactor, in which cells may form large three-dimensional aggregates as they proliferate in the interspatial fiber space. However, the cells growth and performance are significantly inhibited by the lack nutrients. To mitigate this problem, these bioreactors are made small and are not suitable for large scale manufacturing

Another example of a high-density culture system for anchorage dependent cells is a packed-bed bioreactor system. In this this type of bioreactor, a cell substrate is used to provide a surface for the attachment of adherent cells. Medium is perfused along the surface or through the semi-porous substrate to provide nutrients and oxygen needed for the cell growth. For example, packed bed bioreactor systems that contain a packed bed of support or matrix systems to entrap the cells have been previously disclosed U.S. Patent Nos. 4,833,083; 5,501,971; and 5,510,262. Packed bed matrices usually are made of porous particles as substrates or non-woven microfibers of polymer. Such bioreactors function as recirculation flow-through bioreactors. One of the significant issues with such bioreactors is the non-uniformity of cell distribution inside the packed bed. For example, the packed bed functions as depth filter with cells predominantly trapped at the inlet regions, resulting in a gradient of cell distribution during the inoculation step. In addition, due to random fiber packaging, flow resistance and cell trapping efficiency of cross sections of the packed bed are not uniform. For example, medium flows fast though the regions with low cell packing density and flows slowly through the regions where resistance is higher due to higher number of entrapped cells. This creates a channeling effect where nutrients and oxygen are delivered more efficiently to regions with lower volumetric cells densities and regions with higher cell densities are being maintained in suboptimal culture conditions.

Another significant drawback of packed bed systems disclosed in a prior art is the inability to efficiently harvest intact viable cells at the end of culture process. Harvesting of cells is important if the end product is cells, or if the bioreactor is being used as part of a "seed train," where a cell population is grown in one vessel and then transferred to another vessel for further population growth. U.S. Patent No. 9,273,278 discloses a bioreactor design to improve the efficiency of cell recovery from the packed bed during cells harvesting step. It is based on loosening the packed bed matrix and agitation or stirring of packed bed particles to allow porous matrices to collide and thus detach the cells. However, this approach is laborious and may cause significant cells damage, thus reducing overall cell viability.

An example of a packed-bed bioreactor currently on the market is the iCellis® by produced by Pall Corporation. The iCellis uses small strips of cell substrate material consisting of randomly oriented fibers in a non-woven arrangement.

These strips are packed into a vessel to create a packed bed. However, as with similar solutions on the market, there are drawbacks to this type of packed-bed substrate. Specifically, non-uniform packing of the substrate strips creates visible channels within the packed bed, leading to preferential and non-uniform media flow and nutrient distribution through the packed bed. Studies of the iCellis® have noted a "systemic inhomogeneous distribution of cells, with their number increasing from top to bottom of fixed bed," as well as a "nutrient gradient... leading to restricted cell growth and production," all of which lead to the "unequal distribution of cells [that] may impair transfection efficiency." (Rational plasmid design and bioprocess optimization to enhance recombinant adeno-associated virus (AAV) productivity in mammalian cells. Biotechnol. J. 2016, 11, 290-297). Studies have noted that agitation of the packed bed may improve dispersion, but would have other drawbacks (i.e., "necessary agitation for better dispersion during inoculation and transfection would induce increased shear stress, in turn leading to reduced cell viability." *Id.*). Another study noted of the iCellis® that the uneven distribution of cells makes monitoring of the cell population using biomass sensors difficult ("...if the cells are unevenly distributed, the biomass signal from the cells on the top carriers may not show the general view of the entire bioreactor." Process Development of Adenoviral Vector Production in Fixed Bed Bioreactor: From Bench to Commercial Scale. Human Gene Therapy, Vol. 26, No. 8, 2015).

In addition, because of the random arrangement of fibers in the substrate strips and the variation in packing of strips between one packed bed and another of the iCellis®, it can be difficult for customers to predict cell culture performance, since the substrate varies between cultures. Furthermore, the packed substrate of the iCellis® makes efficiently harvesting cells very difficult or impossible, as it is believed that cells are entrapped by the packed bed.

While manufacturing of viral vectors for early-phase clinical trials is possible with existing platforms, there is a need for a platform that can produce high-quality product in greater numbers in order to reach late-stage commercial manufacturing scale. As discussed above, existing cell culture platforms rely on adherent cell substrates of random and uncontrolled structure or porosity, resulting in nonuniform and even unpredictable substrate structure and performance due to random packing, channeling effects, cell entrapment, etc. Regardless of whether the packed bed substrate matrix is uniform or random, there can nonetheless be difficulties in controlling fluid flow as desired through the packed bed, which leads to difficulties in various aspects of cell culture, including uniform cell seeding, cell growth distribution, cell culture media flow uniformity, and harvesting capability.

There is a need for packed-bed cell culture matrices, systems, and methods that enable culturing of cells in a high-density format, with uniform cell distribution, easily attainable and increased harvesting yields, and controllable packed-bed porosity for improved seeding, culturing, and/or harvesting performance.

## SUMMARY

According to an embodiment of this disclosure, a packed-bed bioreactor system for culturing cells is provided. The system includes: a cell culture vessel comprising an inlet, an outlet, and at least one interior reservoir fluidly connected to and disposed in a fluid pathway between the inlet and the outlet; a cell culture matrix disposed in the reservoir in a wound configuration around a winding axis extending in a direction from the inlet to the outlet, the cell culture matrix comprising a structurally defined substrate comprising a substrate material defining a plurality of pores, the substrate material being configured for adhering cells thereto; and a plurality of permeability zones in the cell culture matrix, each of the plurality of permeability zones comprising an opening in the substrate, the opening being larger than a diameter of any of the plurality of pores, wherein the substrate comprises a length extending in a direction tangential to the winding axis and wherein the plurality of permeability zones comprises a variable periodicity along a length of the substrate in the wound configuration. As used herein, "periodicity" refers to the frequency of occurrence, and "variable periodicity" refers to a change in the frequency of the occurrence over a distance or length of the substrate. For example, the plurality of permeability zones having a variable periodicity along a length of the substrate refers to the frequency of permeability zones changing along a length of the substrate, or the density of permeability zones in terms of number of permeability zones per unit length of the substrate changing along the length of the substrate. An increasing periodicity refers to the permeability zones increasing in frequency over a distance, while a decreasing periodicity refers to the permeability zones decreasing in frequency over a distance.

According to additional embodiments of this disclosure, a fixed-bed cell culture matrix for culturing adherent-based cells is provided. The cell culture matrix includes a structurally defined substrate comprising a substrate material defining a plurality of pores, the substrate material being configured for adhering cells thereto, and the substrate material being arranged in a wound configuration; and a plurality of permeability zones in the cell culture matrix, each of the plurality of permeability zones comprising an opening in the substrate, the opening being larger than a diameter of any of the plurality of pores, wherein the substrate material is wound about a winding axis, and the substrate comprises a length extending in a direction tangential to the winding axis.

Additional aspects of the present disclosure will be set forth, in part, in the detailed description, figures and any claims which follow, and in part will be derived from the detailed description, or can be learned by practice of the disclosure. It is to be understood that both the foregoing general description and the following detailed description are exemplary and explanatory only and are not restrictive of the disclosure as disclosed.

## BRIEF DESCRIPTION OF THE DRAWINGS

Figure 1A shows a perspective view of a three-dimensional model of a cell culture substrate, according to one or more embodiments of this disclosure.
Figure 1B is a two-dimensional plan view of the substrate of Figure 1A.
Figure 1C is a cross-section along line A-A of the substrate in Figure 1B.
Figure 2A shows an example of a cell culture substrate, according to some embodiments.
Figure 2B shows an example of a cell culture substrate, according to some embodiments.
Figure 2C shows an example of a cell culture substrate, according to some embodiments.
Figure 3A shows a perspective view of a multilayer cell culture substrate, according to one or more embodiments.
Figure 3B shows a plan view of a multilayer cell culture substrate, according to one or more embodiments.
Figure 4 shows a cross-section view along line B-B of the multilayer cell culture substrate of Figure 3B, according to one or more embodiments.
Figure 5 shows a cross-section view along line C-C of the multilayer cell culture substrate of Figure 4, according to one or more embodiments.
Figure 6 shows a schematic view of a cell culture system, according to one or more embodiments.
Figure 7 shows a cell culture matrix in a rolled cylindrical configuration, according to one or more embodiments.
Figure 8 shows a cell culture system incorporated a rolled cylindrical cell culture matrix, according to one or more embodiments.
Figure 9A shows an unwound substrate material with a plurality of openings forming permeability zones, according to embodiments.
Figure 9B shows the substrate from Figure 9A in a wound configuration, according to embodiments.
Figure 9C shows the substrate from Figures 9A and 9B wound around a core, according to embodiments.

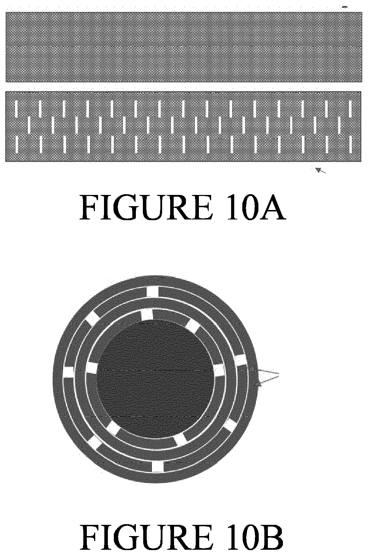

Figure 10A shows a pair of substrates to be wound together, where on has openings to form permeability zones and one does not, according to embodiments.
Figure 10B shows the substrates from Figure 10A when wound around a core, according to embodiments.
Figure 11A shows a substrate with permeability openings of a first periodicity along the length of the substrate, according to embodiments.
Figure 11B shows a substrate with permeability openings of a second periodicity along the length of the substrate, according to embodiments.
Figure 11C shows a substrate with permeability openings of a variable periodicity along the length of the substrate, according to embodiments.
Figure 11D shows a substrate with permeability openings arranged at an angle, according to embodiments.
Figure 12 is a schematic representation of a cell culture system, according to one or more embodiments.

## DETAILED DESCRIPTION

Various embodiments of the disclosure will be described in detail with reference to drawings, if any. Reference to various embodiments does not limit the scope of the invention, which is limited only by the scope of the claims attached hereto. Additionally, any examples set forth in this specification are not limiting and merely set forth some of the many possible embodiments of the claimed invention.

Embodiments of this disclosure packed-bed cell culture substrates, as well as cell culture or bioreactor systems incorporating such packed-bed substrates, and methods of culturing cells using such packed-bed substrates and bioreactor systems.

In conventional large-scale cell culture bioreactors, different types of packed bed bioreactors have been used. Usually these packed beds contain porous substrates or matrices to retain adherent or suspension cells, and to support growth and proliferation. Packed-bed matrices provide high surface area to volume ratios, so cell density can be higher than in the other systems. However, the packed bed often functions as a depth filter, where cells are physically trapped or entangled in fibers of the matrix. Thus, because of linear flow of the cell inoculum through the packed bed, cells are subject to heterogeneous distribution inside the packed bed, leading to variations in cell density through the depth or width of the packed bed. For example, cell density may be higher at the inlet region of a bioreactor and significantly lower nearer to the outlet of the bioreactor. This non-uniform distribution of the cells inside of the packed bed significantly hinders scalability and predictability of such bioreactors in bioprocess manufacturing, and can even lead to reduced efficiency in terms of growth of cells or viral vector production per unit surface area or volume of the packed bed.

Another problem encountered in packed-bed bioreactors disclosed in prior art is the channeling effect. Due to random nature of packed nonwoven fibers, the local fiber density at any given cross section of the packed bed is not uniform. Medium flows quickly in the regions with low fiber density (high bed permeability) and much slower in the regions of high fiber density (lower bed permeability). The resulting non-uniform media perfusion across the packed bed creates the channeling effect, which manifests itself as significant nutrient and metabolite gradients that negatively impact overall cell culture and bioreactor performance. Cells located in the regions of low media perfusion will starve and very often die from the lack of nutrients or metabolite poisoning. Cell harvesting is yet another problem encountered when bioreactors packed with non-woven fibrous scaffolds are used. Due to the packed bed functioning as a depth filter, cells that are released at the end of the cell culture process are entrapped inside the packed bed, and cell recovery is very low. This significantly limits utilization of such bioreactors in bioprocesses where live cells are the products. Thus, the non-uniformity leads to areas with different exposure to flow and shear, effectively reducing the usable cell culture area, causing non-uniform culture, and interfering with transfection efficiency and cell release.

To address these and other problems of existing cell culture solutions, embodiments of the present disclosure provide cell growth substrates, packed-bed matrices of such substrates, and/or packed-bed systems using such substrates that enable efficient and high-yield cell culturing for anchorage-dependent cells and production of cell products (e.g., proteins, antibodies, viral particles). Embodiments include a porous cell-culture matrix made from an ordered and regular array of porous substrate material that enables uniform cell seeding and media/nutrient perfusion, as well as efficient cell harvesting. Embodiments also enable scalable cell-culture solutions with substrates and bioreactors capable of seeding and growing cells and/or harvesting cell products from a process development scale to a full production size scale, without sacrificing the uniform performance of the embodiments. For example, in some embodiments, a bioreactor can be easily scaled from process development scale to product scale with comparable viral genome per unit surface area of substrate ($VG/cm^2$) across the production scale. The harvestability and scalability of the embodiments herein enable their use in efficient seed trains for growing cell populations at multiple scales on the same cell substrate. In addition, the embodiments herein provide a cell culture matrix having a high surface area that, in combination with the other features described, enables a high yield cell culture solution. In some embodiments, for example, the cell culture substrate and/or bioreactors discussed herein can produce $10^{16}$ to $10^{18}$ viral genomes (VG) per batch. Embodiments also include packed-bed cell culture matrices with controlled zonal porosity and defined virtual channels through the packed bed as a way to control the overall performance of the packed bed in terms of fluid flow through the cell culture matrix. This ability to form zones in the packed bed with different porosity will improve, for example, cell seeding uniformity, media perfusion uniformity, cell culture and growth uniformity, and cell harvest.

In one embodiment, a matrix is provided with a structurally defined surface area for adherent cells to attach and proliferate that has good mechanical strength and forms a highly uniform multiplicity of interconnected fluidic networks when assembled in a packed bed or other bioreactor. As used herein, "structurally defined" means that a substrate or matrix having a defined and ordered structure as opposed to a random and disordered structure. Non-woven substrates are considered random and/or disordered, for example. In a structurally defined matrix or substrate, the porosity, fiber size and orientation, and even orientation of separate pieces of substrate material can be designed and controlled.

In particular embodiments, a mechanically stable, non-degradable woven mesh can be used as the substrate to support adherent cell production. The cell culture matrix disclosed herein supports attachment and proliferation of anchorage dependent cells in a high volumetric density format. Uniform cell seeding of such a matrix is achievable, as well as efficient harvesting of cells or other products of the bioreactor. In addition, the embodiments of this disclosure support cell culturing to provide uniform cell distribution during the inoculation step and achieve a confluent monolayer or multilayer of adherent cells on the disclosed matrix, and can avoid formation of large and/or uncontrollable 3D cellular aggregates with limited nutrient diffusion and increased metabolite concentrations. Thus, the matrix eliminates diffusional limitations during operation of the bioreactor. In addition, the matrix enables easy and efficient cell harvest from the bioreactor. The structurally defined matrix of one or more embodiments enables complete cell recovery and consistent cell harvesting from the packed bed of the bioreactor.

According to some embodiments, a method of cell culturing is also provided using bioreactors with the matrix for bioprocessing production of therapeutic proteins, antibodies, viral vaccines, or viral vectors.

In contrast to existing cell culture substrates used in cell culture bioreactors (i.e., non-woven substrates of randomly ordered fibers), embodiments of this disclosure include a structurally defined cell culture substrate having a defined and ordered structure. The defined and order structure allows for consistent and predictable cell culture results. In addition, the substrate has an open porous structure that prevents cell entrapment and enables uniform flow through the packed bed. This construction enables improved cell seeding, nutrient delivery, cell growth, and cell harvesting. According to one or more particular embodiments, the matrix is formed with a substrate material having a thin, sheet-like construction having first and second sides separated by a relatively small thickness, such that the thickness of the sheet is small relative to the width and/or length of the first and second sides of the substrate. In addition, a plurality of holes or openings are formed through the thickness of the substrate. The substrate material between the openings is of a size and geometry that allows cells to adhere to the surface of the substrate material as if it were approximately a two-dimensional (2D) surface, while also allowing adequate fluid flow around the substrate material and through the openings. In some embodiments, the substrate is a polymer-based material, and can be formed as a molded polymer sheet; a polymer sheet with openings punched through the thickness; a number of filaments that are fused into a mesh-like layer; a 3D-printed substrate; or a plurality of filaments that are woven into a mesh layer. The physical structure of the matrix has a high surface-to-volume ratio for culturing anchorage dependent cells. According to various embodiments, the matrix can be arranged or packed in a bioreactor in certain ways discussed here for uniform cell seeding and growth, uniform media perfusion, and efficient cell harvest.

Embodiments of this disclosure can achieve viral vector platforms of a practical size that can produce viral genomes on the scale of greater than about $10^{14}$ viral genomes per batch, greater than about $10^{15}$ viral genomes per batch, greater than about $10^{16}$ viral genomes per batch, greater than about $10^{17}$ viral genomes per batch, or up to or greater than about g $10^{16}$ viral genomes per batch. In some embodiments, production is about $10^{15}$ to about $10^{18}$ or more viral genomes per batch. For example, in some embodiments, the viral genome yield can be about $10^{15}$ to about $10^{16}$ viral genomes or batch, or about $10^{16}$ to about $10^{19}$ viral genomes per batch, or about $10^{16}$-$10^{18}$ viral genomes per batch, or about $10^{17}$ to about $10^{19}$ viral genomes per batch, or about $10^{18}$ to about $10^{19}$ viral genomes per batch, or about $10^{18}$ or more viral genomes per batch.

In addition, the embodiments disclosed herein enable not only cell attachment and growth to a cell culture substrate, but also the viable harvest of cultured cells. The inability to harvest viable cells is a significant drawback in current platforms, and it leads to difficulty in building and sustaining a sufficient number of cells for production capacity. According to an aspect of embodiments of this disclosure, it is possible to harvest viable cells from the cell culture substrate, including between 80% to 100% viable, or about 85% to about 99% viable, or about 90% to about 99% viable. For example, of the cells that are harvested, at least 80% are viable, at least 85% are viable, at least 90% are viable, at least 91% are viable, at least 92% are viable, at least 93% are viable, at least 94% are viable, at least 95% are viable, at least 96% are viable, at least 97% are viable, at least 98% are viable, or at least 99% are viable. Cells may be released from the cell culture substrate using, for example, trypsin, TrypLE, or Accutase.

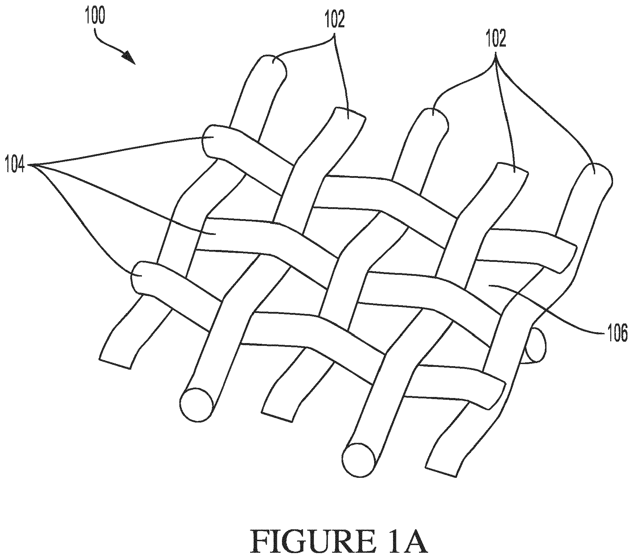

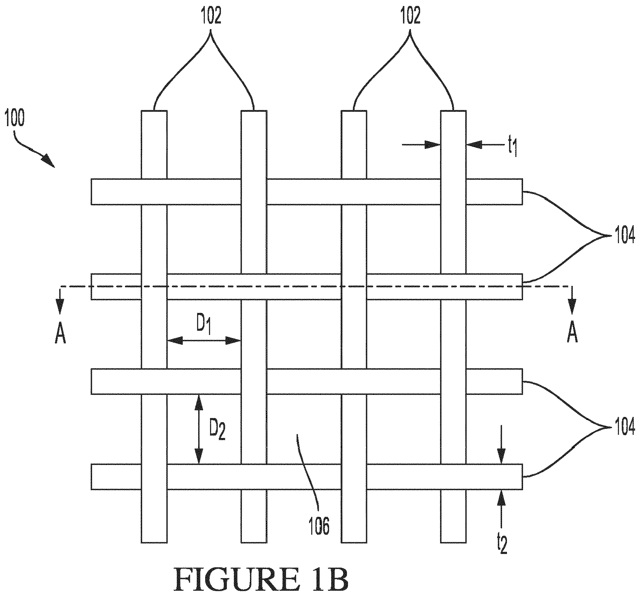

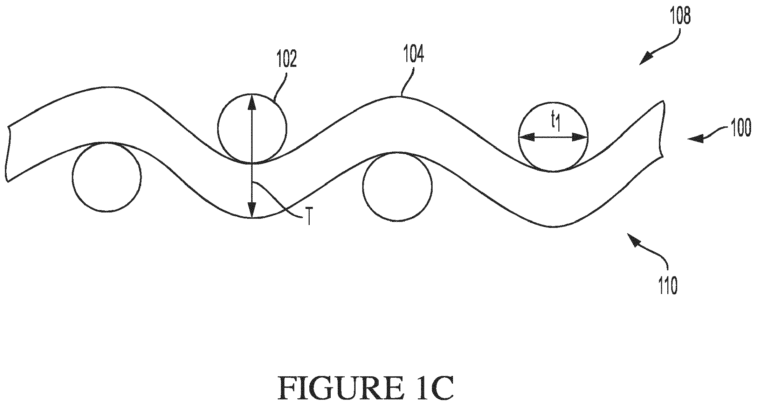

Figures 1A and 1B show a three-dimensional (3D) perspective view and a two-dimensional (2D) plan view, respectively, of a cell culture substrate 100, according to an example of one or more embodiments of this disclosure. The cell culture substrate 100 is a woven mesh layer made of a first plurality of fibers 102 running in a first direction and a second plurality of fibers 104 running in a second direction. The woven fibers of the substrate 100 form a plurality of openings 106, which can be defined by one or more widths or diameters (e.g., $D_1$, $D_2$). The size and shape of the openings can vary based on the type of weave (e.g., number, shape and size of filaments; angle between intersecting filaments, etc.). A woven mesh may be characterized as, on a macro-scale, a two-dimensional sheet or layer. However, a close inspection of a woven mesh reveals a three-dimensional structure due to the rising and falling of intersecting fibers of the mesh. Thus, as shown in Figure 1C, a thickness T of the woven mesh 100 may be thicker than the thickness of a single fiber (e.g., ti). As used herein, the thickness T is the maximum thickness between a first side 108 and a second side 110 of the woven mesh. Without wishing to be bound by theory, it is believed that the three-dimensional structure of the substrate 100 is advantageous as it provides a large surface area for culturing adherent cells, and the structural rigidity of the mesh can provide a consistent and predictable cell culture matrix structure that enables uniform fluid flow.

In Figure 1B, the openings 106 have a diameter $D_1$, defined as a distance between opposite fibers 102, and a diameter $D_2$, defined as a distance between opposite fibers 104. $D_1$ and $D_2$ can be equal or unequal, depending on the weave geometry. Where $D_1$ and $D_2$ are unequal, the larger can be referred to as the major diameter, and the smaller as the minor diameter. In some embodiments, the diameter of an opening may refer to the widest part of the opening. Unless otherwise specified, the opening diameter, as used herein, will refer to a distance between parallel fibers on opposite sides of an opening.

A given fiber of the plurality of fibers 102 has a thickness ti, and a given fiber of the plurality of fibers 104 has a thickness $t_2$. In the case of fibers of round cross-section, as shown in Figure 1A, or other three-dimensional cross-sections, the thicknesses $t_1$ and $t_2$ are the maximum diameters or thicknesses of the fiber cross-section. According to some embodiments, the plurality of fibers 102 all have the same thickness ti, and the plurality of fiber 104 all have the same thickness $t_2$. In addition, $t_1$ and $t_2$ may be equal. However, in one or more embodiments, $t_1$ and $t_2$ are not equal such as when the plurality of fibers 102 are different from the plurality of fiber 104. In addition, each of the plurality of fibers 102 and plurality of fibers 104 may contain fibers of two or more different thicknesses (e.g., $t_{1a}$, $t_{1b}$, etc., and $t_{2a}$, $t_{2b}$, etc.). According to embodiments, the thicknesses $t_1$ and $t_2$ are large relative to the size of the cells cultured thereon, so that the fibers provide an approximation of a flat surface from the perspective of the cell, which can enable better cell attachment and growth as compared to some other solutions in which the fiber size is small (e.g., on the scale of the cell diameter). Due to three-dimensional nature of woven mesh, as shown in Figures 1A-1C, the 2D surface area of the fibers available for cell attachment and proliferation exceeds the surface area for attachment on an equivalent planar 2D surface.

In one or more embodiments, a fiber may have a diameter in a range of about 50 $\mu$m to about 1000 $\mu$m; about

100 μm to about 750 μm; about 125 μm to about 600 μm; about 150 μm to about 500 μm; about 200 μm to about 400 μm; about 200 μm to about 300 μm; or about 150 μm to about 300 μm. On a microscale level, due to the scale of the fiber compared to the cells (e.g., the fiber diameters being larger than the cells), the surface of monofilament fiber is presented as an approximation of a 2D surface for adherent cells to attach and proliferate. Fibers can be woven into a mesh with openings ranging from about 100 μm × 100 μm to about 1000 μm × 1000 μm. In some embodiments, the opening may have a diameter of about 50 μm to about 1000 μm; about 100 μm to about 750 μm; about 125 μm to about 600 μm; about 150 μm to about 500 μm; about 200 μm to about 400 μm; or about 200 μm to about 300 μm. These ranges of the filament diameters and opening diameters are examples of some embodiments, but are not intended to limit the possible feature sizes of the mesh according to all embodiments. The combination of fiber diameter and opening diameter is chosen to provide efficient and uniform fluid flow through the substrate when, for example, the cell culture matrix comprises a number of adjacent mesh layers (e.g., a stack of individual layers or a rolled mesh layer).

Factors such as the fiber diameter, opening diameter, and weave type/pattern will determine the surface area available for cell attachment and growth. In addition, when the cell culture matrix includes a stack, roll, or other arrangement of overlapping substrate, the packing density of the cell culture matrix will impact the surface area of the packed bed matrix. Packing density can vary with the packing thickness of the substrate material (e.g., the space needed for a layer of the substrate). For example, if a stack of cell culture matrix has a certain height, each layer of the stack can be said to have a packing thickness determined by dividing the total height of the stack by the number of layers in the stack. The packing thickness will vary based on fiber diameter and weave, but can also vary based the alignment of adjacent layers in the stack. For instance, due to the three-dimensional nature of a woven layer, there is a certain amount of interlocking or overlapping that adjacent layers can accommodate based on their alignment with one another. In a first alignment, the adjacent layers can be tightly nestled together, but in a second alignment, the adjacent layers can have zero overlap, such as when the lower-most point of the upper layer is in direct contact with the upper-most point of the lower layer. It may be desirable for certain applications to provide a cell culture matrix with a lower density packing of layers (e.g., when higher permeability is a priority) or a higher density of packing (e.g., when maximizing substrate surface area is a priority). According to one or more embodiments, the packing thickness can be from about 50 μm to about 1000 μm; about 100 μm to about 750 μm; about 125 μm to about 600 μm; about 150 μm to about 500 μm; about 200 μm to about 400 μm; about 200 μm to about 300 μm.

The above structural factors can determine the surface area of a cell culture matrix, whether of a single layer of cell culture substrate or of a cell culture matrix having multiple layers of substrate). For example, in a particular embodiment, a single layer of woven mesh substrate having a circular shape and diameter of 6 cm can have an effective surface area of about 68 cm$^2$. The "effective surface area," as used herein, is the total surface area of fibers in a portion of substrate material that is available for cell attachment and growth. Unless stated otherwise, references to "surface area" refer to this effective surface area. According to one or more embodiments, a single woven mesh substrate layer with a diameter of 6 cm may have an effective surface area of about 50 cm$^2$ to about 90 cm$^2$; about 53 cm$^2$ to about 81 cm$^2$; about 68 cm$^2$; about 75 cm$^2$; or about 81 cm$^2$. These ranges of effective surface area are provided for example only, and some embodiments may have different effective surface areas. The cell culture matrix can also be characterized in terms of porosity, as discussed in the Examples herein.

The substrate mesh can be fabricated from monofilament or multifilament fibers of polymeric materials compatible in cell culture applications, including, for example, polystyrene, polyethylene terephthalate, polycarbonate, polyvinylpyrrolidone, polybutadiene, polyvinylchloride, polyethylene oxide, polypyrroles, and polypropylene oxide. Mesh substrates may have a different patterns or weaves, including, for example knitted, warp-knitted, or woven (e.g., plain weave, twilled weave, dutch weave, five needle weave).

The surface chemistry of the mesh filaments may need to be modified to provide desired cell adhesion properties. Such modifications can be made through the chemical treatment of the polymer material of the mesh or by grafting cell adhesion molecules to the filament surface. Alternatively, meshes can be coated with thin layer of biocompatible hydrogels that demonstrate cell adherence properties, including, for example, collagen or Matrigel®. Alternatively, surfaces of filament fibers of the mesh can be rendered with cell adhesive properties through the treatment processes with various types of plasmas, process gases, and/or chemicals known in the industry. In one or more embodiments, however, the mesh is capable of providing an efficient cell growth surface without surface treatment.

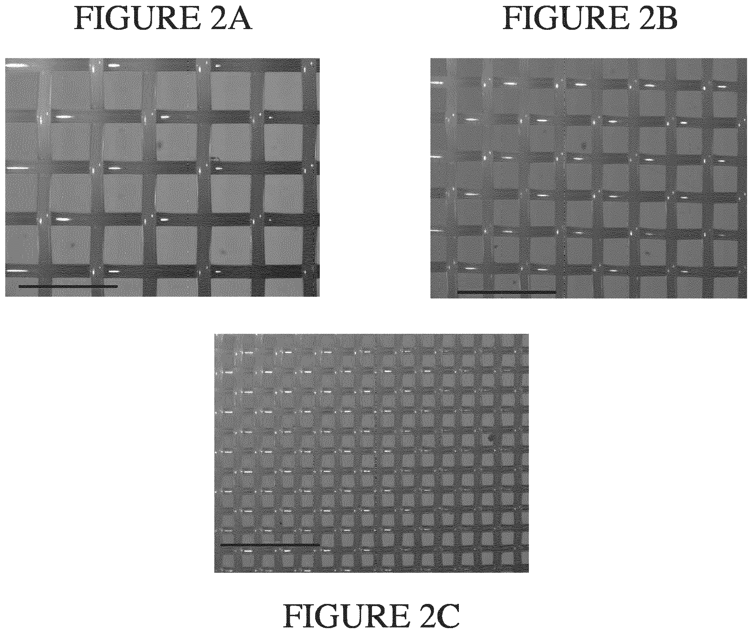

Figures 2A-2C show different examples of woven mesh according to some contemplated embodiments of this disclosure. The fiber diameter and opening size of these meshes are summarized in Table 1 below, as well as the approximate magnitude of increase in cell culture surface area provided by a single layer of the respective meshes relative to a comparable 2D surface. In Table 1, Mesh A refers to the mesh of Figure 2A, Mesh B to the mesh of Figure 2B, and Mesh C to the mesh of Figure 2C. The three mesh geometries of Table 1 are examples only, and embodiments of this disclosure are not limited to these specific examples. Because Mesh C offers the highest surface area, it may be advantageous in achieving a high density in cell adhesion and proliferation, and thus provide the most efficient substrate for cell culturing. However, in some embodiments, it may be advantageous for the cell culture matrix to include a mesh with lower surface area, such as Mesh A or Mesh B, or a combination of meshes of different surface areas, to achieve a desired cell distribution or flow characteristics within the culture chamber, for example.

Table 1. Comparison of meshes in Figures 2A-2C, and the resulting increase in cell culture surface area as compared to a 2D surface.

| | Mesh A | Mesh B | Mesh C |
|---|---|---|---|
| Fiber diameter | 273±3 μm | 218±3 μm | 158±3 μm |
| Mesh opening | 790 x 790 μm | 523 x 523 μm | 244 x 244 μm |
| Surface area increase of one layer of mesh compared to 2D surface | ×1.6 | ×1.8 | ×2.5 |

As shown by the above table, the three-dimensional quality of the meshes provides increased surface area for cell attachment and proliferation compared to a planar 2D surface of comparable size. This increased surface area aids in the scalable performance achieved by embodiments of this disclosure. For process development and process validation studies, small-scale bioreactors are often required to save on reagent cost and increase experimental throughput. Embodiments of this disclosure are applicable to such small-scale studies, but can be scaled-up to industrial or production scale, as well. For example, if 100 layers of Mesh C in the form of 2.2 cm diameter circles are packed into a cylindrical packed bed with a 2.2 cm internal diameter, the total surface area available for cells to attach and proliferate is equal to about 935 cm$^2$. To scale such bioreactor ten times, one could use a similar setup of a cylindrical packed bed with 7 cm internal diameter and 100 layers of the same mesh. In such a case, the total surface area would be equal 9,350 cm$^2$. In some embodiments, the available surface area is about 99,000 cm$^2$/L or more. Because of the plug-type perfusion flow in a packed bed, the same flow rate expressed in ml/min/cm$^2$ of cross-sectioned packed bed surface area can be used in smaller-scale and larger-scale versions of the bioreactor. A larger surface area allows for higher seeding density and higher cell growth density. According to one or more embodiments, the cell culture substrate described herein has demonstrated cell seeding densities of up to 22,000 cells/cm$^2$ or more. For reference, the Corning HyperFlask® has a seeding density on the order of 20,000 cells/cm$^2$ on a two-dimensional surface.

Another advantage of the higher surface areas and higher cell seeding or growing densities is that the cost of the embodiments disclosed herein can be the same or less than competing solutions. Specifically, the cost per cellular product (e.g., per cell or per viral genome) can be equal to or less than other packed-bed bioreactors.

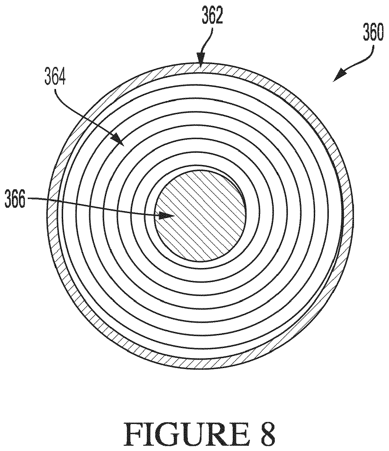

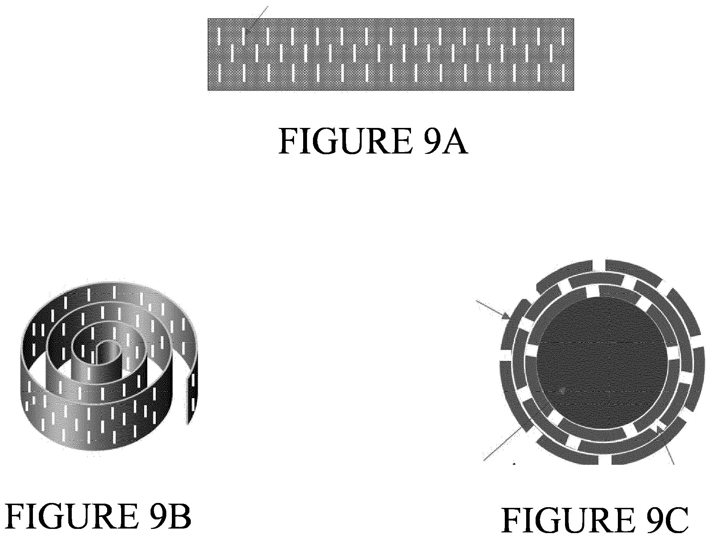

In a further embodiment of the present disclosure discussed below, a woven mesh substrate can be packed in a cylindrical roll format within the bioreactor (see Figures 8 and 9). In such an embodiment, the scalability of the packed bed bioreactor can be achieved by increasing the overall length of the mesh strip and its height. The amount of mesh used in this cylindrical roll configuration can vary based on the desired packing density of the packed bed. For example, the cylindrical rolls can be densely packed in a tight roll or loosely packed in a loose roll. The density of packing will often be determined by the required cell culture substrate surface area required for a given application or scale. In one embodiment, the required length of the mesh can be calculated from the packed bed bioreactor diameter by using following formula:

$$L = \frac{\pi\left(R^2 - r^2\right)}{t} \qquad \text{Equation 1}$$

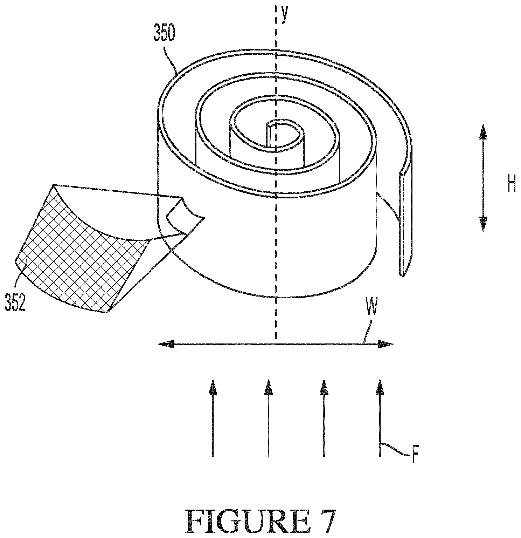

where **L** is the total length of mesh required to pack the bioreactor (i.e., H in Figure 7), **R** is the internal radius of packed bed culture chamber, **r** is the radius of an inner support (support 366 in Figure 8) around which mesh is rolled, and **t** is the thickness of one layer of the mesh. In such a configuration, scalability of the bioreactor can be achieved by increasing diameter or width (i.e., W in Figure 7) of the packed bed cylindrical roll and/or increasing the height **H** of the packed bed cylindrical roll, thus providing more substrate surface area for seeding and growing adherent cells.

By using a structurally defined cell culture matrix of sufficient rigidity, high-flow-resistance uniformity across the matrix or packed bed is achieved. According to various embodiments, the matrix can be deployed in monolayer or multilayer formats. This flexibility eliminates diffusional limitations and provides uniform delivery of nutrients and oxygen to cells attached to the matrix. In addition, the open matrix lacks any cell entrapment regions in the packed bed configuration, allowing for complete cell harvest with high viability at the end of culturing. The matrix also delivers packaging uniformity for the packed bed, and enables direct scalability from process development units to large-scale industrial bioprocessing unit. The ability to directly harvest cells from the packed bed eliminates the need of resuspending a matrix in a stirred or mechanically shaken vessel, which would add complexity and can inflict harmful shear stresses on the cells. Further, the high packing density of the cell culture matrix yields high bioprocess productivity in volumes manageable at the industrial scale.

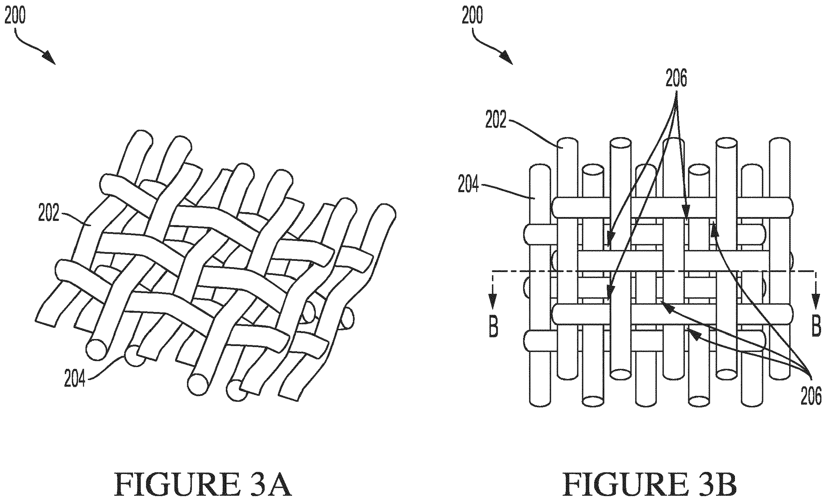

Figure 3A shows an embodiment of the matrix with a multilayer substrate 200, and Figure 3B is a plan view of the same multilayer substrate 200. The multilayer substrate 200 includes a first mesh substrate layer 202 and a second mesh substrate layer 204. Despite the overlapping of the first and second substrate layers 202 and 204, the mesh geometries (e.g., ratio of opening diameters to fiber diameters) is such that the openings of the first and second substrate layers 202 and 204 overlap and provide paths for fluid to flow through the total thickness of the multilayer substrate 200, as shown by the filament-free openings 206 in Figure 3B.

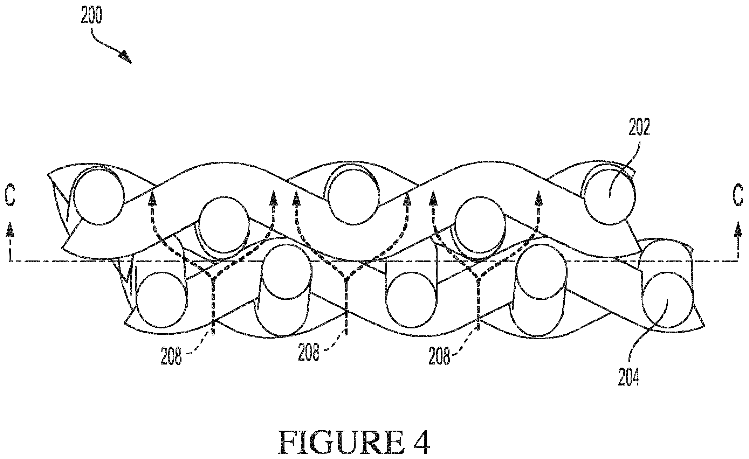

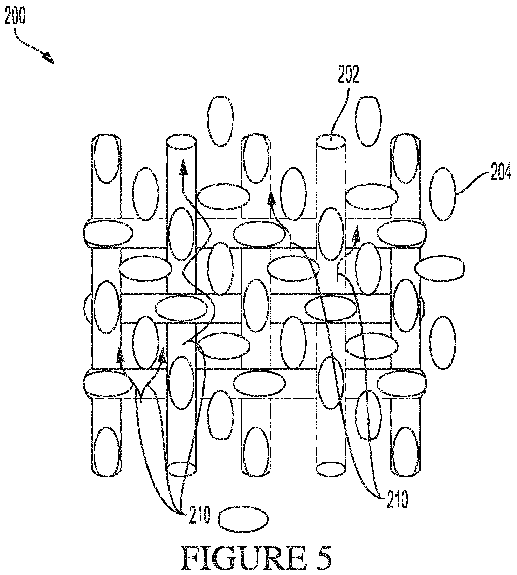

Figure 4 shows a cross section view of the multilayer substrate 200 at line B-B in Figure 3B. The arrows 208 show the possible fluid flow paths through openings in the second substrate layer 204 and then around filaments in the first substrate layer 202. The geometry of the mesh substrate layers is designed to allow efficient and uniform flow through one or multiple substrate layers. In addition, the structure of the matrix 200 can accommodate fluid flow through the matrix in multiple orientations. For example, as shown in Figure 4, the direction of bulk fluid flow (as shown by arrows 208) is perpendicular to the major side surfaces of the first and second substrate layers 202 and 204. However, the matrix can also be oriented with respect to the flow such that the sides of the substrate layers are parallel to the bulk flow direction. Figure 5 shows a cross section view of the multilayer substrate 200 along line C-C in Figure 4, and the structure of matrix 200 allows for fluid flow (arrows 210) through fluid pathways in the multilayer substrate 200. In addition to fluid flow being perpendicular or parallel to the first and second sides of the mesh layers, the matrix can be arranged with multiple pieces of substrate at intermediate angles, or even in random arrangements with respect to fluid flow. This flexibility in orientation is enabled by the essentially isotropic flow behavior of the woven substrate. In contrast, substrates for adherent cells in existing bioreactors do not exhibit this behavior and instead their packed beds tend to create preferential flow channels and have substrate materials with anisotropic permeability. The flexibility of the matrix of the current disclosure allows for its use in various applications and bioreactor or container designs while enabling better and more uniform permeability throughout the bioreactor vessel.

As discussed herein, the cell culture substrate can be used within a bioreactor vessel, according to one or more embodiments. For example, the substrate can be used in a packed bed bioreactor configuration, or in other configurations within a three-dimensional culture chamber. However, embodiments are not limited to a three-dimensional culture space, and it is contemplated that the substrate can be used in what may be considered a two-dimensional culture surface configuration, where the one or more layers of the substrate lay flat, such as within a flat-bottomed culture dish, to provide a culture substrate for cells. Due to contamination concerns, the vessel can be a single-use vessel that can be disposed of after use.

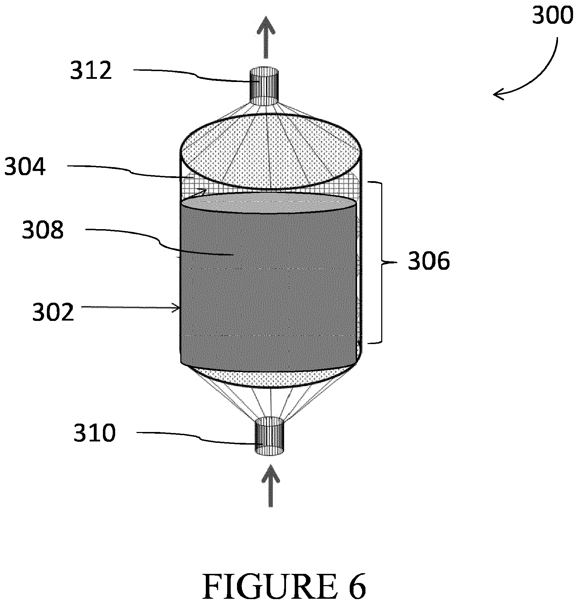

A cell culture system is provided, according to one or more embodiments, in which the cell culture matrix is used within a culture chamber of a bioreactor vessel. Figure 6 shows an example of a cell culture system 300 that includes a bioreactor vessel 302 having a cell culture chamber 304 in the interior of the bioreactor vessel 302. Within the cell culture chamber 304 is a cell culture matrix 306 that is made from a stack of substrate layers 308. The substrate layers 308 are stacked with the first or second side of a substrate layer facing a first or second side of an adjacent substrate layer. The bioreactor vessel 300 has an inlet 310 at one end for the input of media, cells, and/or nutrients into the culture chamber 304, and an outlet 312 at the opposite end for removing media, cells, or cell products from the culture chamber 304. By allowing stacking of substrate layers in this way, the system can be easily scaled up without negative impacts on cell attachment and proliferation, due to the defined structure and efficient fluid flow through the stacked substrates. While the vessel 300 may generally be described as having an inlet 310 and an outlet 312, some embodiments may use one or both of the inlet 310 and outlet 312 for flowing media, cells, or other contents both into and out of the culture chamber 304. For example, inlet 310 may be used for flowing media or cells into the culture chamber 304 during cell seeding, perfusion, or culturing phases, but may also be used for removing one or more of media, cells, or cell products through the inlet 310 in a harvesting phase. Thus, the terms "inlet" and "outlet" are not intended to restrict the function of those openings.

In one or more embodiments, flow resistance and volumetric density of the packed bed can be controlled by interleaving substrate layers of different geometries. In particular, mesh size and geometry (e.g., fiber diameter, opening diameter, and/or opening geometry) define the fluid flow resistance in packed bed format. By interlaying meshes of different sizes and geometries, flow resistance can be controlled or varied in one or more specific portions of the bioreactor. This can enable better uniformity of liquid perfusion in the packed bed. For example, 10 layers of Mesh A (Table 1) followed by 10 layers of Mesh B (Table 1) and followed by 10 layers of Mesh C (Table 1) can be stacked to achieve a desired packed bed characteristic. As another example, the packed bed may start with 10 layers of Mesh B, followed by 50 layers of Mesh C, followed by 10 layers of Mesh B. Such repetition pattern may continue until the full bioreactor is packed with mesh. These are examples only, and used for illustrative purposes without intending to be limiting on the possible combinations. Indeed, various combinations of meshes of different sizes are possible to obtain different profiles of volumetric density of cells growth surface and flow resistance. For example, a packed bed column with zones of varying volumetric cells densities (e.g., a series of zones creating a pattern of low/high/low/high, etc. densities) can be assembled by interleaving meshes of different sizes. As discussed below, zones of variable porosity can also be provided by creating channels through all or part of the packed bed.

In Figure 6, the bulk flow direction is in a direction from the inlet 310 to the outlet 312, and, in this example, the first and second major sides of the substrate layers 308 are parallel to the bulk flow direction. However, embodiments are not limited to this configuration, and the substate can be arranged perpendicular to the bulk flow direction, or at any angle thereto. In some embodiments, the substrate is sized and shaped to fill the interior space defined by the culture chamber so that the culture spaces in each vessel are filled for cell growth surfaces to maximize efficiency in terms of cells per unit volume. According to various embodiments herein, distribution plates can be used to help distribute the media, cells, or nutrients across a cross-section of the packed bed and thus improve uniformity of fluid flow through the packed bed. As such, the multiple inlets 330 represent how a distribution plate can be provided with a plurality of holes across the packed-bed cross-section for creating more uniform flow.

Figure 7 shows an embodiment of the matrix in which the substrate is formed into a cylindrical roll 350. For example, a sheet of a matrix material that includes a mesh substrate 352 is rolled into a cylinder about a central longitudinal axis y. The cylindrical roll 350 has a width W along a dimension perpendicular to the central longitudinal axis y and a height H along a direction perpendicular to the central longitudinal axis y. In one or more preferred embodiments, the cylindrical roll 350 is designed to be within a bioreactor vessel such that the central longitudinal axis y is parallel to a direction of bulk flow F of fluid through the bioreactor or culture chamber that houses the cylindrical roll. Figure 8 shows a cell culture system 360 having a bioreactor vessel 362 that houses a cell culture matrix 364 in such a cylindrical roll configuration. Like the cylindrical roll 350 in Figure 7, the cell culture matrix 364 has a central longitudinal axis, which, in Figure 8, extends into the page. The system 360 further includes a central support member 366 around which the cell culture matrix 364 is position. The central support member 366 can be provided purely for physical support and/or alignment of the cell culture matrix 364, but can also provide other functions, according to some embodiments. For example, the central support member 366 can be provided with one or more openings for supplying media to the cell culture matrix 364 along the length H of the matrix. In other embodiments, the central support member 366 may include one or more attachment sites for holding one or more portions of the cell culture matrix 364 at the inner part of the cylindrical roll. These attachment sites may be hooks, clasps, posts, clamps, or other means of attaching the mesh sheet to the central support member 366.

As discussed above, packed-bed cell culture matrices such as those shown in Figures 3A-9 can provide many performance advantages due to the uniform, consistent, and/or predictable structurally defined substrate of the matrix. In some embodiments disclosed herein, such cell culture matrices have consistent porosity and structure across the width and/or height of the packed bed. For example, Figure 6, each of the substrate layers 308 can have a uniform and substantially identical structure (e.g., a woven disk with an un-broken weave across the substrate layer 308). This helps achieve consistent and uniform fluid flow throughout the packed bed. However, in some cases, it may be desirable to vary the porosity and structure of all or part of a packed bed to control the fluid flow therethrough in desired ways. Accordingly, embodiments of this disclosure include packed-bed matrices with one or more defined permeability zones having porosities and/or permeabilities that differ from the porosity or permeability of other regions of the packed-bed matrix. As an aspect of some of these embodiments, virtual channels are created to increase flow in specific areas of the cell culture matrix, thus creating these permeability zones.

As used herein, "permeability zone" is defined as a portion of a cell culture matrix where the cell culture substrate is altered to increase permeability or porosity of the matrix in a defined area relative to a portion of the cell culture matrix containing only unaltered cell culture substrate. The cell culture matrix is considered "altered" when the otherwise regular defined and ordered structure of the cell culture substrate is interrupted to increase permeability or porosity in the altered location relative to the unaltered location. Such alternation can be achieved by removal of portions of the substrate material (e.g., by cutting out substrate material; gaps molded into the substrate material; controlled dissolution of portions of a dissolvable substrate; or vacancies left in a substrate material made by any other method, including 3D printing). Although the term "altered" may imply temporality wherein the substrate material undergoes a change from its original form, it should be understood that, within the context of this disclosure, "altered" can mean any variation of the defined and regular structure of the cell culture substrate, whether that variation is created when the substrate material itself is created or that variation is achieved by changing the substrate material after the substrate material is already created. In other words, the alteration results in an opening in the substrate material or matrix that is larger than an average pore size of the structurally defined cell culture substrate of the matrix. An "unaltered" area of cell culture substrate should be understood to mean any portion of a cell culture substrate having the defined and ordered structure of the structurally defined substrate, without any alterations or interruptions of that structure. Due to the permeability zone being the result of altered portions of cell culture substrate, permeability zones are differentiated from local variations in permeability due to non-uniform or randomly packed substrates in existing packed-bed systems discussed above.

As used herein, a "virtual channel" is defined as a fluid flow pathway through the matrix that has higher local permeability than a portion of the matrix containing only unaltered cell culture substrate. Such channels are described as "virtual" because they channels are not physically constrained (e.g., by walls or tubing), but rather exist within the cell culture matrix, which has an open porous structure. Thus, there is not necessarily any barrier separating a virtual channel from the remainder of the cell culture matrix. A virtual channel may be considered to extend in a horizontal (i.e., perpendicular to a bulk flow direction of media through the cell culture matrix or bioreactor) cross-section of the matrix, or it may be considered to extend longitudinally (i.e., parallel to the bulk flow direction) through part of or the entirety of the packed-bed matrix.

"Permeability zones" and "virtual channels" as used herein can contain areas of both altered and unaltered substrate material. For example, embodiments of this disclosure include multi-layered packed-bed cell culture matrices having stacked layers of cell culture substrate. A permeability zone or virtual channel can be created in the stacked layers by removing substrate material from a vertical section of the packed-bed, and all or only a portion of the layers in the vertical section may have material removed to increase permeability. For example, even if only a fraction of the layers in the vertical section have material removed, the permeability through that vertical section can be higher than through another vertical section of the matrix containing only unaltered substrate layers.

According to embodiments of this disclosure, the permeability zones will exhibit higher permeability relative to the other portions of the cell culture matrix. As discussed above, the permeability zones can contain one or more openings in the cell culture matrix that results in a higher than normal permeability in those zones. The permeability zones can extend along the extent of the height h of the cell culture matrix, but this does not necessarily mean that the entirety of the zones are open and unobstructed. Rather, the permeability zones may include only one or select areas where portions of the cell culture matrix have an opening for increased permeability. However, the permeability zones can still be considered to extend along the extent of the height h because any reduction in flow resistance within those zones can result in increased permeability within the zones, along the flow direction F.

The use of defined permeability zones to control permeability in a packed-bed cell culture matrix can have many uses. For example, depending on the type or stage of cell culture, it may desirable to achieve a particular permeability profile. One use of the permeability zones is to compensate for non-uniform flow properties of a bioreactor system. That is, where a bioreactor system inherently has non-uniform flow of media or fluid, permeability zones in the packed bed can be used to compensate for that non-uniformity and can result in a composite flow profile that is more uniform.

In some embodiments, the opening of the virtual channel can have a width of from about 200 $\mu$m to about 3000 $\mu$m, from about 300 $\mu$m to about 2500 $\mu$m, from about 400 $\mu$m to about 2000 $\mu$m, from about 500 $\mu$m to about 1500 $\mu$m, from about 600 $\mu$m to about 1800 $\mu$m, from about 1000 $\mu$m to about 2000 $\mu$m, from about 1000 $\mu$m to about 1800 $\mu$m, or from about 1300 $\mu$m to about 1400 $\mu$m. Also, as discussed above, the shape of the virtual channels can vary a great deal, and so too can the dimension of the opening.

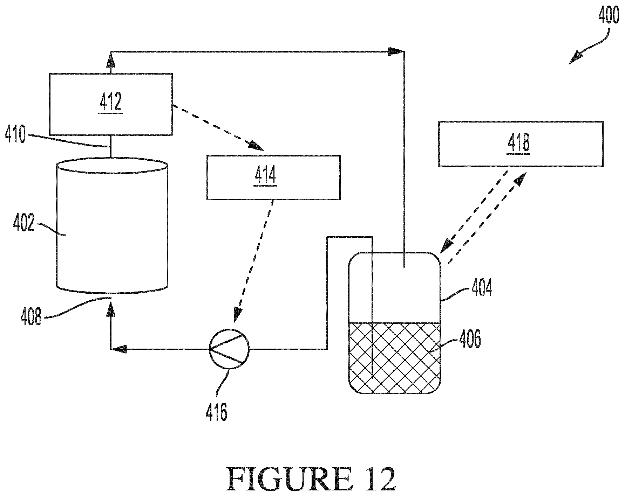

Figure 12 shows a cell culture system 500 according to one or more embodiments. The system 500 includes a bioreactor 502 housing the cell culture matrix of one or more embodiments disclosed herein. The bioreactor 502 can be fluidly connected to a media conditioning vessel 504, and the system is capable of supplying a cell culture media 506 within the conditioning vessel 504 to the bioreactor 502. The media conditioning vessel 504 can include sensors and control components found in typical bioreactor used in the bioprocessing industry for a suspension batch, fed-batch or perfusion culture. These include but are not limited to DO oxygen sensors, pH sensors, oxygenator/gas sparging unit, temperature probes, and nutrient addition and base addition ports. A gas mixture supplied to sparging unit can be controlled by a gas flow controller for $N_2$, $O_2$, and $CO_2$ gasses. The media conditioning vessel 504 also contains an impeller for media mixing. All media parameters measured by sensors listed above can be controlled by a media conditioning control unit 518 in communication with the media conditioning vessel 504, and capable of measuring and/or adjusting the conditions of the cell culture media 506 to the desired levels. As shown in Figure 12, the media conditioning vessel 504 is provided as a vessel that is separate from the bioreactor vessel 502. This can have advantages in terms of being able to condition the media separate from where the cells are cultured, and then supplying the conditioned media to the cell culture space. However, in some embodiments, media conditioning can be performed within the bioreactor vessel 502.

The media from the media 506 conditioning vessel 504 is delivered to the bioreactor 502 via an inlet 508, which may also include an injection port for cell inoculum to seed and begin culturing of cells. The bioreactor vessel 502 may also include on or more outlets 510 through which the cell culture media 506 exits the vessel 502. In addition, cells or cell products may be output through the outlet 510. To analyze the contents of the outflow from the bioreactor 502, one or more sensors 512 may be provided in the line. In some embodiments, the system 500 includes a flow control unit 514 for controlling the flow into the bioreactor 502. For example, the flow control unit 514 may receive a signal from the one or more sensors 512 (e.g., an $O_2$ sensor) and, based on the signal, adjust the flow into the bioreactor 502 by sending a signal to a pump 516 (e.g., peristaltic pump) upstream of the inlet 508 to the bioreactor 502. Thus, based on one or a combination of factors measured by the sensors 512, the pump 516 can control the flow into the bioreactor 502 to obtain the desired cell culturing conditions.

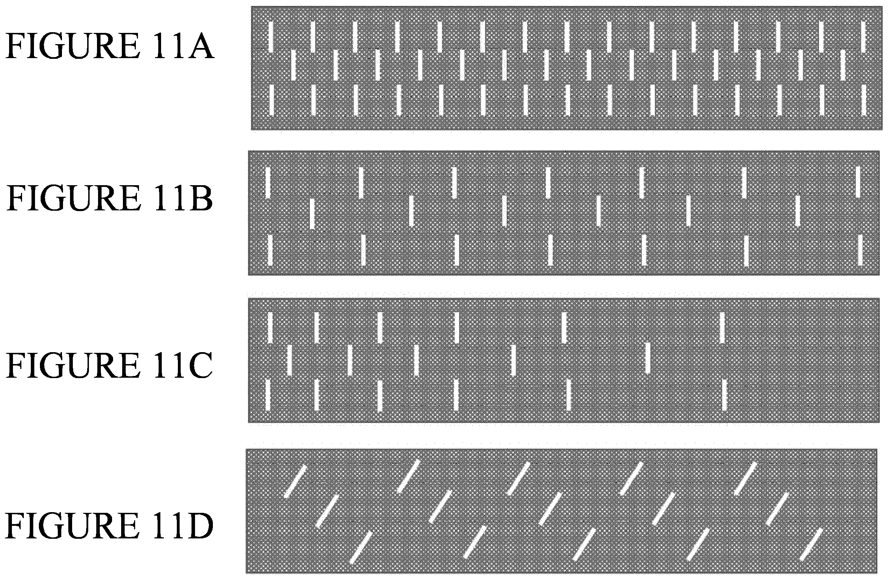

The media perfusion rate is controlled by the signal processing unit 514 that collects and compares sensors signals from media conditioning vessel 504 and sensors located at the packed bed bioreactor outlet 510. Because of the pack flow nature of media perfusion through the packed bed bioreactor 502, nutrients, pH and oxygen gradients are developed along the packed bed. The perfusion flow rate of the bioreactor can be automatically controlled by the flow control unit 514 operably connected to the peristaltic pump 516, according to the flow chart in Figure 11.

One or more embodiments of this disclosure offer a cell inoculation step that is different from conventional methods. In conventional methods, a pack bed with a conventional matrix is filled with culture media and concentrated inoculum is injected into the media circulation loop. The cell suspension is pumped through the bioreactor at increased flow rate to reduce nonuniformity of cell seeding via capture on the conventional packed bed matrix. In such conventional methods, the pumping of cells in the circulation loop at an elevated flow rate continues for perhaps several hours until the majority of the cells are captured in packed bed bioreactor. However, because of the nonuniform deep bed filtration nature of conventional packed bed bioreactors, cells are distributed nonuniformly inside the packed bed with the higher cell density at the inlet region of the bioreactor and lower cell density at the outlet region of the bioreactor.

In contrast, according to embodiments of the present disclosure, cell inoculum of equal volume to the void volume of the culture chamber in the bioreactor is directly injected into the packed bed through a cell inoculum injection port at the inlet 508 of the bioreactor 502 (Figure 12). The cell suspension is then uniformly distributed inside the packed bed because of uniform and continuous fluidic passages present in the cell culture matrix described herein. To prevent cells sedimentation due to gravity forces at the initial seeding stage, media perfusion can be started immediately after the inoculum injection. The perfusion flow rate is maintained below a preprogrammed threshold to balance the force of gravity and to avoid cells being washed from the packed bed bioreactor. Thus, at the initial cell attachment stage, cells are gently tumbled inside the packed bed and uniform cells distribution and attachment on available substrate surface is achieved.

The cell culture matrix can be arranged in multiple configurations within the culture chamber depending on the desired system. For example, in one or more embodiments, the system includes one or more layers of the substrate with a width extending across the width of a defined cell culture space in the culture chamber. Multiple layers of the substrate may be stacked in this way to a predetermined height. As discussed above, the substrate layers may be arranged such that the first and second sides of one or more layers are perpendicular to a bulk flow direction of culture media through the defined culture space within the culture chamber, or the first and second sides of one or more layers may be parallel to the bulk flow direction. In one or more embodiments, the cell culture matrix includes one or more substrate layers at a first orientation with respect to the bulk flow, and one or more other layers at a second orientation that is different from the first orientation. For example, various layers may have first and second sides that are parallel or perpendicular to the bulk flow direction, or at some angle in between.

In one or more embodiments, the cell culture system includes a plurality of discrete pieces of the cell culture substrate in a packed bed configuration, where the length and or width of the pieces of substrate are small relative to the culture chamber. As used herein, the pieces of substrate are considered to have a length and/or width that is small relative to the culture chamber when the length and/or width of the piece of substrate is about 50% or less of the length and/or width of the culture space. Thus, the cell culture system may include a plurality of pieces of substrate packed into the culture space in a desired arrangement. The arrangement of substrate pieces may be random or semi-random, or may have a predetermined order or alignment, such as the pieces being oriented in a substantially similar orientation (e.g., horizontal, vertical, or at an angle between 0° and 90° relative to the bulk flow direction).

The "defined culture space," as used herein, refers to a space within the culture chamber occupied by the cell culture matrix and in which cell seeding and/or culturing is to occur. The defined culture space can fill approximately the entirety of the culture chamber, or may occupy a portion of the space within the culture chamber. As used herein, the "bulk flow direction" is defined as a direction of bulk mass flow of fluid or culture media through or over the cell culture matrix during the culturing of cells, and/or during the inflow or outflow of culture media to the culture chamber.

In one or more embodiments, the cell culture matrix is secured within the culture chamber by a fixing mechanism. The fixing mechanism may secure a portion of the cell culture matrix to a wall of the culture chamber that surrounds the matrix, or to a chamber wall at one end of the culture chamber. In some embodiments, the fixing mechanism adheres a portion of the cell culture matrix to a member running through the culture chamber, such as member running parallel to the longitudinal axis of the culture chamber, or to a member running perpendicular to the longitudinal axis. However, in one or more other embodiments, the cell culture matrix may be contained within the culture chamber without being fixedly attached to the wall of the chamber or bioreactor vessel. For example, the matrix may be contained by the boundaries of the culture chamber or other structural members within the chamber such that the matrix is held within a predetermined area of the bioreactor vessel without the matrix being fixedly secured to those boundaries or structural members.

One aspect of some embodiments provides a bioreactor vessel in a roller bottle configuration. The culture chamber is capable of containing a cell culture matrix and substrate according to one or more of the embodiments described in this disclosure. In the roller bottle configuration, the bioreactor vessel may be operably attached to a means for moving the bioreactor vessel about a central longitudinal axis of the vessel. For example, the bioreactor vessel may be rotated about the central longitudinal axis. The rotation may be continuous (e.g., continuing in one direction) or discontinuous (e.g., an intermittent rotation in a single direction or alternating directions, or oscillating in back and forth rotational directions). In operation, the rotation of the bioreactor vessel causes movement of cells and/or fluid within the chamber. This movement can be considered relative with respect to the walls of the chamber. For example, as the bioreactor vessel rotates about its central longitudinal axis, gravity may cause the fluid, culture media, and/or unadhered cells to remain toward a lower portion of the chamber. However, in one or more embodiments, the cell culture matrix is essentially fixed with respect to the vessel, and thus rotates with the vessel. In one or more other embodiments, the cell culture matrix can be unattached and free to move to a desired degree relative to the vessel as the vessel rotates. The cells may adhere to the cell culture matrix, while the movement of the vessel allows the cells to receive exposure to both the cell culture media or liquid, and to oxygen or other gases within the culture chamber.

By using a cell culture matrix according to embodiments of this disclosure, such as a matrix including a woven or mesh substrate, the roller bottle vessel is provided with an increased surface area available for adherent cells to attach, proliferate, and function. In particular, using a substrate of a woven mesh of monofilament polymer material within the roller bottle, the surface area may increase by of about 2.4 to about 4.8 times, or to about 10 times that of a standard roller bottle. As discussed herein, each monofilament strand of the mesh substrate is capable of presenting itself as 2D surface for adherent cells to attach. In addition, multiple layers of mesh can we arranged in roller bottle, resulting in increases of total available surface area ranging from about 2 to 20 times that of a standard roller bottle. Thus, existing roller bottle facilities and processing, including cell seeding, media exchange, and cell harvesting, can be modified by the addition of the improved cell culture matrix disclosed herein, with minimal impact on existing operation infrastructure and processing steps.

The bioreactor vessel optionally includes one or more outlets capable of being attached to inlet and/or outlet means. Through the one or more outlets, liquid, media, or cells can be supplied to or removed from the chamber. A single port in the vessel may act as both the inlet and outlet, or multiple ports may be provided for dedicated inlets and outlets.

The packed bed cell culture matrix of one or more embodiments can consist of the woven cell culture mesh substrate without any other form of cell culture substrate disposed in or interspersed with the cell culture matrix. That is, the woven cell culture mesh substrate of embodiments of this disclosure are effective cell culture substrates without requiring the type of irregular, non-woven substrates used in existing solution. This enables cell culture systems of simplified design and construction, while providing a high-density cell culture substrate with the other advantages discussed herein related to flow uniformity, harvestability, etc.

As discussed herein, the cell culture substrates and bioreactor systems provided offer numerous advantages. For example, the embodiments of this disclosure can support the production of any of a number of viral vectors, such as AAV (all serotypes) and lentivirus, and can be applied toward in vivo and ex vivo gene therapy applications. The uniform cell seeding and distribution maximizes viral vector yield per vessel, and the designs enable harvesting of viable cells, which can be useful for seed trains consisting of multiple expansion periods using the same platform. In addition, the embodiments herein are scalable from process development scale to production scale, which ultimately saves development time and cost. The methods and systems disclosed herein also allow for automation and control of the cell culture process to maximize vector yield and improve reproducibility. Finally, the number of vessels needed to reach production-level scales of viral vectors (e.g., $10^{16}$ to $10^{18}$ AAV VG per batch) can be greatly reduced compared to other cell culture solutions.

Embodiments are not limited to the vessel rotation about a central longitudinal axis. For example, the vessel may rotate about an axis that is not centrally located with respect to the vessel. In addition, the axis of rotation may be a horizonal or vertical axis.

Illustrative Implementations

The following is a description of various aspects of implementations of the disclosed subject matter. Each aspect may include one or more of the various features, characteristics, or advantages of the disclosed subject matter. The implementations are intended to illustrate a few aspects of the disclosed subject matter and should not be considered a comprehensive or exhaustive description of all possible implementations.

Aspect 1 pertains to a fixed-bed bioreactor system for culturing cells, the system comprising: a cell culture vessel comprising an inlet, an outlet, and at least one interior reservoir fluidly connected to and disposed in a fluid pathway between the inlet and the outlet; a cell culture matrix disposed in the reservoir in a wound configuration around a winding axis extending in a direction from the inlet to the outlet, the cell culture matrix comprising a structurally defined substrate comprising a substrate material defining a plurality of pores, the substrate material being configured for adhering cells thereto; and a plurality of permeability zones in the cell culture matrix, each of the plurality of permeability zones comprising an opening in the substrate, the opening being larger than a diameter of any of the plurality of pores, wherein the substrate comprises a length extending in a direction tangential to the winding axis, and wherein the plurality of permeability zones comprises a variable periodicity along a length of the substrate in the wound configuration.

Aspect 2 pertains to the fixed-bed bioreactor system of Aspect 1, wherein each of the plurality of permeability zones comprises a higher permeability than a standard permeability of the cell culture matrix outside of the at least one permeability zone.

Aspect 3 pertains to the fixed-bed bioreactor system of Aspect 1, wherein the periodicity decreases along the length from a first end of the substrate material, the first end being at the interior of the wound configuration of the cell culture matrix.

Aspect 4 pertains to the fixed-bed bioreactor system of Aspect 1, wherein the periodicity increases along the length from a first end of the substrate material, the first end being at the interior of the wound configuration of the cell culture matrix.

Aspect 5 pertains to the fixed-bed bioreactor system of Aspect 1, wherein the cell culture matrix comprises a plurality of layers of the substrate material that are wound together in the wound configuration.

Aspect 6 pertains to the fixed-bed bioreactor system of Aspect 5, wherein each of the plurality of layers of the substate material comprises one or more permeability zones comprising an opening.

Aspect 7 pertains to the fixed-bed bioreactor system of Aspect 5, wherein at least one of the plurality of layers of the substate material does not comprise a permeability zone.

Aspect 8 pertains to the fixed-bed bioreactor system of Aspect 5, wherein the plurality of layers of the substrate comprises one or more layers comprising a permeability zone and one or more layers comprising no permeability zones in an alternating arrangement.

Aspect 9 pertains to the fixed-bed bioreactor system of Aspect 1, wherein the openings of the permeability zones have a length and a width, the length being longer than the width.

Aspect 10 pertains to the fixed-bed bioreactor system of Aspect 9, wherein the length of the openings extends in a direction parallel to a direction from the inlet to the outlet.

Aspect 11 pertains to the fixed-bed bioreactor system of Aspect 9, wherein the length of the openings extends in a direction that is not parallel to a direction from the inlet to the outlet.

Aspect 12 pertains to the fixed-bed bioreactor system of Aspect 11, wherein the length of the opening extends in a direction that is perpendicular to a direction from the inlet to the outlet.

Aspect 13 pertains to the fixed-bed bioreactor system of Aspect 11, wherein the length of the opening extends in a direction that is between 0° and 90° relative to a direction from the inlet to the outlet.

Aspect 14 pertains to the fixed-bed bioreactor system of Aspect 13, wherein the length of the opening extends in a direction that is 45° relative to a direction from the inlet to the outlet.

Aspect 15 pertains to the fixed-bed bioreactor system of Aspect 1, wherein a thickness of the substate material bordering the openings of the permeability zones is greater than a thickness of the substrate material not bordering the openings.

Aspect 16 pertains to the fixed-bed bioreactor system of Aspect 1, wherein the substrate material comprises an ordered and substantially uniform array of pores.

Aspect 17 pertains to the fixed-bed bioreactor system of Aspect any one of Aspects 1-16, wherein the permeability zones of the cell culture matrix are designed to compensate for at least one of an increased permeability between the cell culture matrix and a wall of the reservoir and an increased permeability within a core of the wound cell culture matrix.

Aspect 18 pertains to the fixed-bed bioreactor system of any one of Aspects 2-17, wherein the standard permeability is a permeability of a section of the cell culture matrix consisting of the substrate without the opening.

Aspect 19 pertains to the fixed-bed bioreactor system of any one of Aspects 1-18, wherein the opening comprises a width of from about 10 $\mu$m to about 3000 $\mu$m, from about 300 $\mu$m to about 2500 $\mu$m, from about 400 $\mu$m to about 2000 $\mu$m, from about 500 $\mu$m to about 1500 $\mu$m, from about 600 $\mu$m to about 1800 $\mu$m, from about 1000 $\mu$m to about 2000 $\mu$m, from about 1000 $\mu$m to about 1800 $\mu$m, from about 1300 $\mu$m to about 1400 $\mu$m, or from about 10 $\mu$m to about 100 $\mu$m.

Aspect 20 pertains to the fixed-bed cell culture matrix for culturing adherent-based cells in a bioreactor, the cell culture matrix comprising: a structurally defined substrate comprising a substrate material defining a plurality of pores, the substrate material being configured for adhering cells thereto, and the substrate material being arranged in a wound configuration; and a plurality of permeability zones in the cell culture matrix, each of the plurality of permeability zones comprising an opening in the substrate, the opening being larger than a diameter of any of the plurality of pores, wherein the substrate material is wound about a winding axis, and the substrate comprises a length extending in a direction tangential to the winding axis.

Aspect 21 pertains to the fixed-bed cell culture matrix of Aspect 20, wherein the plurality of permeability zones comprises a variable periodicity along a length of the substrate in the wound configuration.

Aspect 22 pertains to the fixed-bed cell culture matrix of Aspect 20, wherein each of the plurality of permeability zones comprises a higher permeability than a standard permeability of the cell culture matrix outside of the at least one permeability zone.

Aspect 23 pertains to the fixed-bed cell culture matrix of Aspect 20, wherein the periodicity decreases along the length from a first end of the substrate material, the first end being at the interior of the wound configuration of the cell culture matrix.

Aspect 24 pertains to the fixed-bed cell culture matrix of Aspect 20, wherein the periodicity increases along the length from a first end of the substrate material, the first end being at the interior of the wound configuration of the cell culture matrix.

Aspect 25 pertains to the fixed-bed cell culture matrix of Aspect 20, wherein the cell culture matrix comprises a plurality of layers of the substrate material that are wound together in the wound configuration.

Aspect 26 pertains to the fixed-bed cell culture matrix of Aspect 25, wherein each of the plurality of layers of the substate material comprises one or more permeability zones comprising an opening.

Aspect 27 pertains to the fixed-bed cell culture matrix of Aspect 25, wherein at least one of the plurality of layers of the substate material does not comprise a permeability zone.

Aspect 28 pertains to the fixed-bed cell culture matrix of Aspect 25, wherein the plurality of layers of the substrate comprises one or more layers comprising a permeability zone and one or more layers comprising no permeability zones in an alternating arrangement.

Aspect 29 pertains to the fixed-bed cell culture matrix of Aspect 20, wherein the openings of the permeability zones have a length and a width, the length being longer than the width.

Aspect 30 pertains to the fixed-bed cell culture matrix of Aspect 29, wherein the length of the openings extends in a direction parallel to the winding axis.

Aspect 31 pertains to the fixed-bed cell culture matrix of Aspect 29, wherein the length of the openings extends in a direction that is not parallel to the winding axis.

Aspect 32 pertains to the fixed-bed cell culture matrix of Aspect 31, wherein the length of the opening extends in a direction that is perpendicular to the winding axis.

Aspect 33 pertains to the fixed-bed cell culture matrix of Aspect 31, wherein the length of the opening extends in a direction that is between 0° and 90° relative to a direction of the winding axis.

Aspect 34 pertains to the fixed-bed cell culture matrix of Aspect 33, wherein the length of the opening extends in a direction that is 45° relative to a direction of the winding axis.

Aspect 35 pertains to the fixed-bed cell culture matrix of Aspect 20, wherein a thickness of the substate material bordering the openings of the permeability zones is greater than a thickness of the substrate material not bordering the openings.

Aspect 36 pertains to the fixed-bed cell culture matrix of Aspect 20, wherein the substrate material comprises an ordered and substantially uniform array of pores.

Aspect 37 pertains to the fixed-bed cell culture matrix of any one of Aspects 22-36, wherein the standard permeability is a permeability of a section of the cell culture matrix consisting of the substrate without the opening.

Aspect 28 pertains to the fixed-bed cell culture matrix of any one of Aspects 20-37, wherein the opening comprises a width of from about 10 $\mu$m to about 3000 $\mu$m, from about 300 $\mu$m to about 2500 $\mu$m, from about 400 $\mu$m to about 2000 $\mu$m, from about 500 $\mu$m to about 1500 $\mu$m, from about 600 $\mu$m to about 1800 $\mu$m, from about 1000 $\mu$m to about 2000 $\mu$m, from about 1000 $\mu$m to about 1800 $\mu$m, from about 1300 $\mu$m to about 1400 $\mu$m, or from about 10 $\mu$m to about 100 $\mu$m.

Definitions

"Wholly synthetic" or "fully synthetic" refers to a cell culture article, such as a microcarrier or surface of a culture vessel, that is composed entirely of synthetic source materials and is devoid of any animal derived or animal sourced materials. The disclosed wholly synthetic cell culture article eliminates the risk of xenogeneic contamination.

"Include," "includes," or like terms means encompassing but not limited to, that is, inclusive and not exclusive.

"Users" refers to those who use the systems, methods, articles, or kits disclosed herein, and include those who are culturing cells for harvesting of cells or cell products, or those who are using cells or cell products cultured and/or harvested according to embodiments herein.

"About" modifying, for example, the quantity of an ingredient in a composition, concentrations, volumes, process temperature, process time, yields, flow rates, pressures, viscosities, and like values, and ranges thereof, or a dimension of a component, and like values, and ranges thereof, employed in describing the embodiments of the disclosure, refers to variation in the numerical quantity that can occur, for example: through typical measuring and handling procedures used for preparing materials, compositions, composites, concentrates, component parts, articles of manufacture, or use formulations; through inadvertent error in these procedures; through differences in the manufacture, source, or purity of starting materials or ingredients used to carry out the methods; and like considerations. The term "about" also encompasses amounts that differ due to aging of a composition or formulation with a particular initial concentration or mixture, and amounts that differ due to mixing or processing a composition or formulation with a particular initial concentration or mixture.

"Optional" or "optionally" means that the subsequently described event or circumstance can or cannot occur, and that the description includes instances where the event or circumstance occurs and instances where it does not.

The indefinite article "a" or "an" and its corresponding definite article "the" as used herein means at least one, or one or more, unless specified otherwise.

Abbreviations, which are well known to one of ordinary skill in the art, may be used (e.g., "h" or "hrs" for hour or hours, "g" or "gm" for gram(s), "mL" for milliliters, and "rt" for room temperature, "nm" for nanometers, and like abbreviations).

Specific and preferred values disclosed for components, ingredients, additives, dimensions, conditions, and like aspects, and ranges thereof, are for illustration only; they do not exclude other defined values or other values within defined ranges. The systems, kits, and methods of the disclosure can include any value or any combination of the values, specific values, more specific values, and preferred values described herein, including explicit or implicit intermediate values and ranges.

Unless otherwise expressly stated, it is in no way intended that any method set forth herein be construed as requiring that its steps be performed in a specific order. Accordingly, where a method claim does not actually recite an order to be followed by its steps or it is not otherwise specifically stated in the claims or descriptions that the steps are to be limited to a specific order, it is in no way intended that any particular order be inferred.

It will be apparent to those skilled in the art that various modifications and variations can be made without departing from the spirit or scope of the disclosed embodiments. Since modifications, combinations, sub-combinations and variations of the disclosed embodiments incorporating the spirit and substance of the embodiments may occur to persons skilled in the art, the disclosed embodiments should be construed to include everything within the scope of the appended claims and their equivalents.

**Claims**

1. A fixed-bed bioreactor system for culturing cells, the system comprising:

   a cell culture vessel comprising an inlet, an outlet, and at least one interior reservoir fluidly connected to and disposed in a fluid pathway between the inlet and the outlet;
   a cell culture matrix disposed in the reservoir in a wound configuration around a winding axis extending in a direction from the inlet to the outlet, the cell culture matrix comprising a structurally defined substrate comprising a substrate material defining a plurality of pores, the substrate material being configured for adhering cells thereto; and
   a plurality of permeability zones in the cell culture matrix, each of the plurality of permeability zones comprising an opening in the substrate, the opening being larger than a diameter of any of the plurality of pores,
   wherein the substrate comprises a length extending in a direction tangential to the winding axis, and
   wherein the plurality of permeability zones comprises a variable periodicity along a length of the substrate in the wound configuration.

2. The fixed-bed bioreactor system of claim 1, wherein each of the plurality of permeability zones comprises a higher permeability than a standard permeability of the cell culture matrix outside of the at least one permeability zone,

3. The fixed-bed bioreactor system of claim 1 or claim 2, wherein the periodicity decreases along the length from a first end of the substrate material, the first end being at the interior of the wound configuration of the cell culture matrix.

4. The fixed-bed bioreactor system of any preceding claim, wherein the periodicity increases along the length from a first end of the substrate material, the first end being at the interior of the wound configuration of the cell culture matrix.

5. The fixed-bed bioreactor system of any preceding claim, wherein the cell culture matrix comprises a plurality of layers of the substrate material that are wound together in the wound configuration.

6. The fixed-bed bioreactor system of claim 5, wherein each of the plurality of layers of the substate material comprises one or more permeability zones comprising an opening.

7. The fixed-bed bioreactor system of claim 5, wherein at least one of the plurality of layers of the substate material does not comprise a permeability zone.

8. The fixed-bed bioreactor system of claim 5, wherein the plurality of layers of the substrate comprises one or more layers comprising a permeability zone and one or more layers comprising no permeability zones in an alternating arrangement.

9. The fixed-bed bioreactor system of any preceding claim, wherein the openings of the permeability zones have a length and a width, the length being longer than the width.

10. The fixed-bed bioreactor system of claim 9, wherein the length of the openings extends in a direction parallel to a direction from the inlet to the outlet.

11. The fixed-bed bioreactor system of claim 9, wherein the length of the openings extends in a direction that is not parallel to a direction from the inlet to the outlet.

12. The fixed-bed bioreactor system of claim 11, wherein the length of the opening extends in a direction that is between 0° and 90° relative to a direction from the inlet to the outlet.

13. The fixed-bed bioreactor system of, wherein a thickness of the substate material bordering the openings of the permeability zones is greater than a thickness of the substrate material not bordering the openings.

14. The packed-bed bioreactor system of any preceding claim, wherein the substrate material comprises an ordered and substantially uniform array of pores.

15. The fixed-bed bioreactor system of any one of claims 1-14, wherein the permeability zones of the cell culture matrix are designed to compensate for at least one of an increased permeability between the cell culture matrix and a wall of the reservoir and an increased permeability within a core of the wound cell culture matrix.

FIGURE 1A

FIGURE 1B

FIGURE 1C

## FIGURE 2A

## FIGURE 2B

## FIGURE 2C

EP 4 424 811 A1

FIGURE 3B

FIGURE 3A

FIGURE 4

FIGURE 5

EP 4 424 811 A1

EP 4 424 811 A1

FIGURE 6

FIGURE 7

FIGURE 8

FIGURE 9A

FIGURE 9C

FIGURE 9B

# FIGURE 10A

# FIGURE 10B

FIGURE 11A

FIGURE 11B

FIGURE 11C

FIGURE 11D

**FIGURE 12**

Europäisches
Patentamt
European
Patent Office
Office européen
des brevets

# EUROPEAN SEARCH REPORT

**Application Number**

EP 24 16 0068

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2022/115305 A1 (CORNING INC [US]) 2 June 2022 (2022-06-02) * paragraph [0093] * * paragraph [0097] - paragraph [0100] * * figures 9,10A-10C,11A-11F * | 1-15 | INV. C12M1/12 |
| A | US 3 941 661 A (NOTEBOOM WILLIAM D) 2 March 1976 (1976-03-02) * column 2, line 31 - column 3, line 7; figure 1 * | 1-15 | |
| A | CN 101 294 136 A (BIOSYSTEM MEDICAL TECHNOLOGY S [CN]) 29 October 2008 (2008-10-29) * the whole document * | 1-15 | |
| A | US 2023/018016 A1 (CHANG KING-MING [TW]) 19 January 2023 (2023-01-19) * paragraph [0065] - paragraph [0069] * * paragraph [0073] * * figures 1-3,5,6 * | 1-15 | |

**TECHNICAL FIELDS SEARCHED (IPC)**

C12M

The present search report has been drawn up for all claims

| Place of search | Date of completion of the search | Examiner |
|---|---|---|
| Berlin | 3 July 2024 | Cubas Alcaraz, Jose |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 16 0068

This annex lists the patent family members relating to the patent documents cited in the above-mentioned European search report.
The members are as contained in the European Patent Office EDP file on
The European Patent Office is in no way liable for these particulars which are merely given for the purpose of information.

03-07-2024

| Patent document cited in search report | | Publication date | Patent family member(s) | | Publication date |
|---|---|---|---|---|---|
| WO 2022115305 | A1 | 02-06-2022 | CN | 116529353 A | 01-08-2023 |
| | | | EP | 4251726 A1 | 04-10-2023 |
| | | | JP | 2024501108 A | 11-01-2024 |
| | | | US | 2024010972 A1 | 11-01-2024 |
| | | | WO | 2022115305 A1 | 02-06-2022 |
| US 3941661 | A | 02-03-1976 | NONE | | |
| CN 101294136 | A | 29-10-2008 | NONE | | |
| US 2023018016 | A1 | 19-01-2023 | CN | 114787333 A | 22-07-2022 |
| | | | EP | 4081629 A1 | 02-11-2022 |
| | | | GB | 2606928 A | 23-11-2022 |
| | | | JP | 2023504607 A | 06-02-2023 |
| | | | KR | 20220092594 A | 01-07-2022 |
| | | | TW | 202124708 A | 01-07-2021 |
| | | | US | 2023018016 A1 | 19-01-2023 |
| | | | WO | 2021129472 A1 | 01-07-2021 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63448907
- US 4833083 A
- US 5501971 A
- US 5510262 A
- US 9273278 B

**Non-patent literature cited in the description**

- Rational plasmid design and bioprocess optimization to enhance recombinant adeno-associated virus (AAV) productivity in mammalian cells. *Biotechnol. J.,* 2016, vol. 11, 290-297
- Process Development of Adenoviral Vector Production in Fixed Bed Bioreactor: From Bench to Commercial Scale. *Human Gene Therapy,* 2015, vol. 26 (8